Europäisches Patentamt

European Patent Office

Office européen des brevets

(11) Publication number: **0 355 993**
**A1**

## EUROPEAN PATENT APPLICATION

(21) Application number: **89307350.2**

(51) Int. Cl.⁴: **B63C 11/12**

(22) Date of filing: **20.07.89**

(30) Priority: **22.07.88 JP 97002/88 U**

(43) Date of publication of application:
**28.02.90 Bulletin 90/09**

(84) Designated Contracting States:
**AT BE CH DE ES FR GB GR IT LI LU NL SE**

(71) Applicant: **Kamitani, Shigeki**
**8-1-933 Nakahara 1-Chome Isogo-ku**
**Yokohama-shi Kanagawa-ken 235(JP)**

(72) Inventor: **Kamitani, Shigeki**
**8-1-933 Nakahara 1-Chome Isogo-ku**
**Yokohama-shi Kanagawa-ken 235(JP)**

(74) Representative: **Leale, Robin George et al**
**FRANK B. DEHN & CO. Imperial House 15-19**
**Kingsway**
**London WC2B 6UZ(GB)**

(54) Face mask for use under water.

(57) A face mask for use under water, which is arranged to sealingly cover at least the eyes and nose of a user, and including conduit means (5a,5b) for sealingly connecting each of the user's ear passages (E) to the interior of the mask, to equalise the air pressure therebetween.

**FIG.1**

## Face mask for use under water

The present invention relates to a face mask for use under water, e.g. in scuba diving.

Scuba divers have an inclination not to use earplugs to close their ears. As a result, water will pass in and out of the ear passages until the pressure within the ear passages and the water pressure balance each other. This may cause bradyacusia. Also, there is a danger of breaking the eardrum. If the ear drum is broken, cold water reaches the three semicircular canals of the inner ear, thereby causing the diver to lose his sense of balance. As a result the diver cannot make a decision as to which is up or down, and this is a dangerous situation. This can be prevented by using earplugs, but during a deep dive the earplugs may be pushed so deep into the ears by the water pressure that it is difficult to remove them. For fear of this, scuba divers prefer not to use earplugs.

Also, when using earplugs, the user cannot hear any sounds under water, and thus cannot have the pleasure of hearing under water sounds or speaking to other people, unless such sounds or voices are very loud.

According to the present invention there is provided a face mask for use under water, which is arranged to sealingly cover at least the eyes and nose of a user, and including conduit means for sealingly connecting each of the user's ear passages to the interior of the mask, to equalise the air pressure therebetween.

Preferably the said conduit means comprises two tubes each having one end communicating with the interior of the mask and the other end adapted to fit sealingly in a respective ear passage.

Preferably the said other end of each said tube is formed as an earpiece provided with a plurality of external annular flanges, spaced along the tube, to make sealing engagement with the ear passage.

In use of such a mask, air from the user's lungs will communicate with the closed interior of the face mask through his nose, and then reach his ears through the said conduit means. The air in the user's lungs is at the same pressure as the water pressure at the depth at which the user is swimming. Therefore the pressure in the ear passages and the water pressure are balanced, no matter how deep the user may dive. Consequently, the ends of the conduit means which are received in the ear passages cannot be collapsed or pushed deep into the ears, thus ensuring that the mask can be used safely.

When sounds are made under water, the sound waves will cause the window of the face mask to resonate, and then the air trapped in the interior of the mask will vibrate accordingly, for the sounds to reach the user's ears via the conduit means. The sound-receiving area of the face mask window can be increased to its limit by directing the window towards the sound source. Thus, such sounds can be heard clearly.

An embodiment of the invention will now be described by way of example and with reference to the accompanying drawings, in which:-

Fig. 1 is a perspective view of a face mask according to the invention; and

Fig. 2 is a longitudinal section of an earpiece of the face mask.

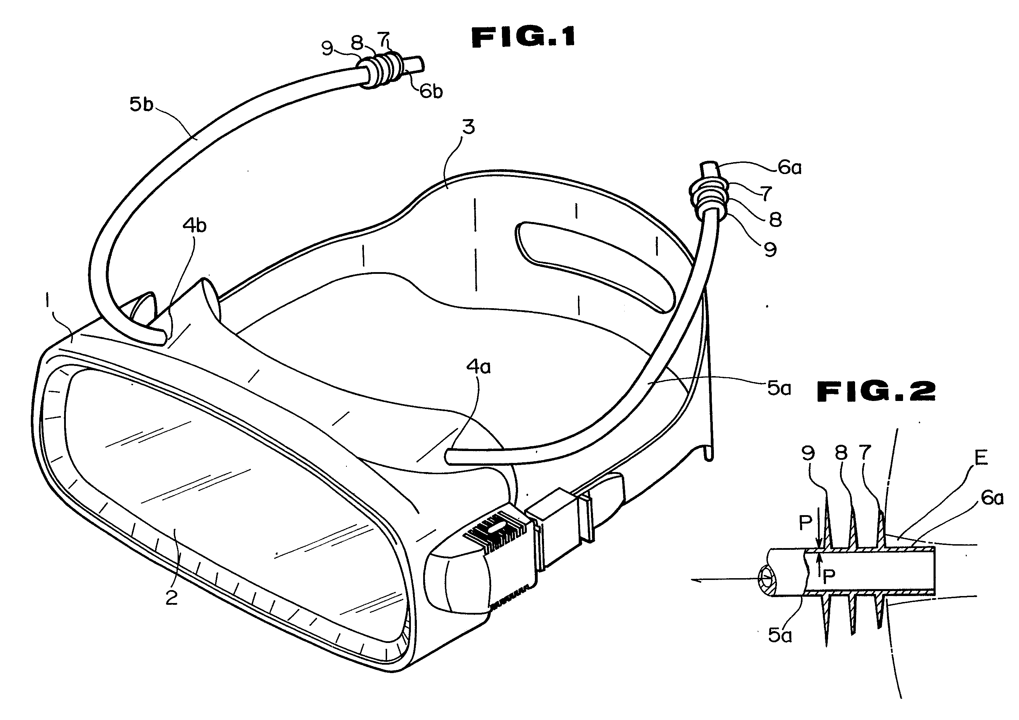

Fig. 1 shows a face mask which is arranged to cover the eyes and nose of a scuba diver, but it should be understood that the present invention can be equally applied to a face mask which is designed to cover the full area of the user's face. In Fig. 1 a mask body 1 has a transparent window 2 made of, for instance, glass. When a scuba diver wears the face mask, it will cover his eyes and nose to define a closed space which is filled with air within the face mask. Straps are indicated at 3.

A pair of tubes 5a and 5b are connected to the opposite sides 4a and 4b of the mask body, and these tubes are long enough to reach the ears of the user to permit his ear passages E to communicate with the space inside the mask body 1. As shown, each tube has an earpiece at its end 6a or 6b.

The tube size may be arbitrarily selected, but its end size should be selected to closely fit in the ear passage E. In this particular embodiment each earpiece has a plurality of annular flanges 7,8 and 9 longitudinally arranged and integrally connected thereto. These annular flanges prevent ingress of water into the ear passage E, and they may have smaller diameters towards the end of the tube.

When a scuba diver wears this face mask to cover his eyes and nose, with its tube ends inserted in his ears, each tube has the same pressure P applied thereto as the water pressure at the depth at which the user is swimming.

The air in the user's lungs communicates with his ear passages through his nose and the tubes 5a and 5b. This air has the same pressure as the water pressure at the depth at which the user is swimming. Therefore, the earpieces 6a and 6b will not be collapsed. They cannot be pushed into the ear passages either, thus ensuring that the earpieces are safe to use. When a sound is produced in the water it will reach the tubes to vibrate the air inside, thereby permitting the user to hear the sound. Also, the sound will reach the window 2 of the face mask to vibrate the air inside. The user can hear the sound well by directing the window towards the sound source, to increase the sound receiving area of the window.

The annular flanges 7,8 and 9 prevent ingress of water into the ear passages of a user, and at the same time they make it easy for the earpieces to fit closely in the ear passages.

A generally "Y"-shaped tube may be used in place of the two tubes 5a and 5b. Also, each earpiece may have a stopper to close its open end when not in use.

It will thus be seen that the present invention, at least in its preferred forms, provides a face mask equipped with earpieces which permit a user to hear clearly sounds made under water or to hear other people speaking while still assuring that the earpieces can be easily removed from the ears after finishing a dive. Thus, scuba diving will be more fun, and users will not suffer from difficulty in hearing, and will not lose their sense of balance while swimming under water.

It is to be clearly understood that there are no particular features of the foregoing specification, or of any claims appended hereto, which are at present regarded as being essential to the performance of the present invention, and that any one or more of such features or combinations thereof may therefore be included in, added to, omitted from or deleted from any of such claims if and when amended during the prosecution of this application or in the filing or prosecution of any divisional application based thereon. Furthermore the manner in which any of such features of the specification or claims are described or defined may be amended, broadened or otherwise modified in any manner which falls within the knowledge of a person skilled in the relevant art, for example so as to encompass, either implicitly or explicitly, equivalents or generalisations thereof.

## Claims

1. A face mask for use under water, which is arranged to sealingly cover at least the eyes and nose of a user, and including conduit means for sealingly connecting each of the user's ear passages to the interior of the mask, to equalise the air pressure therebetween.

2. A face mask as claimed in claim 1, wherein the said conduit means comprises two tubes each having one end communicating with the interior of the mask and the other end adapted to fit sealingly in a respective ear passage.

3. A face mask as claimed in claim 2, wherein the said other end of each said tube is formed as an earpiece provided with a plurality of external annular flanges, spaced along the tube, to make sealing engagement with the ear passage.

4. A face mask as claimed in any preceding claim, which is arranged to cover the whole face of the user.

**FIG.1**

**FIG.2**

EP 0 355 993 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-2 488 235 (PFEIFFER)<br>* Whole document *<br>--- | 1 | B 63 C 11/12 |
| A | GB-A- 800 910 (REEVES)<br>* Page 2, lines 92-107; figure 2 *<br>----- | 1 | |

TECHNICAL FIELDS
SEARCHED (Int. Cl.5)

B 63 C

The present search report has been drawn up for all claims

| Place of search | Date of completion of the search | Examiner |
|---|---|---|
| THE HAGUE | 06-11-1989 | VISENTIN, M. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P0401)